(19) **Europäisches Patentamt**

**European Patent Office**

**Office européen des brevets**

(11) Publication number: **0 589 557 A2**

## EUROPEAN PATENT APPLICATION

(21) Application number: **93306152.5**

(22) Date of filing: **04.08.93**

(51) Int. Cl.5: **B01J 23/02**, C07C 1/20, C07C 11/02, B01J 37/02

(30) Priority: **21.09.92 JP 250854/92**
**10.11.92 JP 299614/92**
**08.04.93 JP 81812/93**

(43) Date of publication of application:
**30.03.94 Bulletin 94/13**

(84) Designated Contracting States:
**DE FR GB IT NL**

(71) Applicant: **SUMITOMO CHEMICAL COMPANY, LIMITED**
**5-33 Kitahama 4-chome**
**Chuo-ku**
**Osaka-shi Osaka(JP)**

(72) Inventor: **Ishino, Masaru**
**1-9-937, Yushudainishi**
**Ichihara-shi, Chiba(JP)**
Inventor: **Nakayama, Toshio**
**6-13-13, Kuranamidai**
**Sodegaura-shi, Chiba(JP)**

(74) Representative: **Diamond, Bryan Clive et al**
**Gee & Co.**
**Chancery House**
**Chancery Lane**
**London WC2A 1OU (GB)**

(54) **Preparation of silica-alumine supported catalysts and of tertiary olefins by means of these catalysts.**

(57) Two methods are disclosed for preparing a silica-alumina supported catalyst (a) silica-alumina, e.g. as pellets, preferably previously calcined at 700-1200 °C, is soaked in an aqueous solution of a hydroxide, or a weak acid salt of an alkali metal (Na,K) or an alkaline earth metal (Mg) whilst the pH is kept at 7-11 (preferably 7-10) so that the solution permeates the Si-Al solids; or

(b) silica, e.g. as pellets, is soaked in an aqueous solution of an aluminum salt or Si powder is mixed with an Al salt, and the Al salt thus supported on Si is heated at a temperature, e.g. 100-1200 °C, below the decomposition temperature of the salt, and the product is contacted with an aqueous solution as defined above, preferably by soaking the SiAl carrier in the solution at a pH of 7 to 11 so that the solution permeates the carrier.

A gaseous tert-alkyl ether (MTBE) is contacted with the catalyst thus prepared at 100-400 °C, optionally with addition of steam, so as to provide a tert-olefin (IB), in good yield and with low by-product formation.

EP 0 589 557 A2

The present invention relates to a process of preparing a silicon-aluminum catalyst and a process for producing a tertiary olefin using the catalyst.

It is known to produce a tertiary olefin by dehydration of an alcohol in the presence of sulfuric acid. However, this method suffers from the problems that sulfuric acid is so corrosive that expensive corrosion-resistant materials are necessary for the equipment, and undesirable side-reactions such as polymerization reactions or hydration reactions occur.

In addition, methods for producing a tertiary olefin by contacting a tert-alkyl ether with a catalyst have been known. The catalysts known to be used in such methods include $\gamma$-alumina (JP-B-47-41882), an alumina modified with a silicon compound (JP-A-51-39604), a metal sulfate (JP-B-51-26401), a silica modified with various kinds of metal ions (JP-A-55-2695), a silica-alumina calcined at high temperature (JP-A-57-28012), a silica-alumina treated with a hydroxide or a weak acid salt of an alkaline metal (JP-A-59-55837 and JP-A-3-220136), and an aluminum salt supported on silica (JP-B-62-48645). The terms "JP-A" and "JP-B" refer to unexamined and examined published Japanese patent applications respectively. However, those conventional methods have the problems that they need a relatively high reaction temperature, and large amounts of undesirable by-products such as a tertiary olefin dimer and a dialkyl ether are produced, and consequently the yields of a tertiary olefin which is the desired product and of an alcohol which is a useful by-product are low.

Accordingly, one object of the present invention is to provide a novel process of producing silicon-aluminum catalysts.

Another object of the present invnetion is to provide a process for producing a tertiary olefin using the catalysts.

A process according to the present invention of preparing a silicon-aluminum catalyst comprises soaking silica-alumina in an aqueous solution of a hydroxide or a weak acid salt of an alkali metal and/or an alkaline earth metal, wherein the pH of the aqueous solution is kept in the range of 7 to 11 throughout the soaking.

A second embodiment of preparing a silicon-aluminum catalyst comprises supporting a hydroxide or a weak acid salt of an alkali metal and/or an alkaline earth metal on a carrier, wherein the carrier is obtained by heating silica having an aluminum salt supported thereon at a temperature higher than the temperature at which the aluminum salt decomposes.

The invention also embraces the catalysts thus produced.

The process for producing a tertiary olefin according to the present invention comprises contacting a gaseous tert-alkyl ether with a catalyst of the first or second embodiment of the present invention.

The latter process has an advantage that the production reaction proceeds at a relatively low temperature, production of undesirable by-products such as a tertiary olefin dimer and a dialkyl either is controlled at a low level and the yields of the tertiary olefin which is the desired product and an alcohol which is a useful by-product are high.

The first embodiment of the preparation of the catalyst is now explained in detail.

As the silica-alumina used for the soaking in the said solution at pH 7 to 11, not only a synthetic silica-alumina prepared by a conventional method but also a silica-alumina containing impurities such as an acid clay and an activated clay can be used. As a form of silica-alumina, any of powder, crushed pieces and molded pellets can be used. Particularly, a molded silica-alumina which is commercially available is preferable. The form of the molded pellets is not limited; e.g. globules or cylinders can be employed. The size thereof is usually 1 to 20 mm, preferably 3 to 6 mm. When the size is too small, the pressure drop throughout a reactor sometimes becomes large and consequently reaction operation becomes difficult; on the other hand, when the size is too large, the efficiency of a reactor becomes low and consequently a large amount of catalyst becomes necessary. Prior to the soaking, the silica-alumina used is preferably calcined in air, inert gas or steam at a temperature in the range of preferably 700 to 1,200°C, more preferably 800 to 1,000°C for usually 0.1 to 24 hours.

Examples of the alkali metals and the alkaline earth metals are sodium, potassium and magnesium, sodium is preferred. Examples of the weak acids are a carbonate, a bicarbonate and an organic acid salt.

It is assumed that the catalyst of the present invention has a suitably controlled acidity, neither too strong nor too weak, which can be obtained by soaking silica-alumina in an aqueous solution of a hydroxide or a weak acid salt of an alkali metal and/or an alkaline earth metal, wherein the pH of the aqueous solution is kept in the aforesaid specific range throughout the soaking. Control of the pH of the aqueous solution throughout the soaking is particularly important; the pH must be kept in the range of 7 to 11, preferably 7 to 10, throughout the soaking. When the pH is too low or too high, the controlling of the catalyst activity is insufficient and consequently the yield of a tertiary olefin becomes low because of increase of undesirable by-products such as a tertiary olefin dimer and a dialkyl ether. Since the pH of the aqueous solution tends

2

to become lower during the soaking, it is necessary to add a solution of the alkali metal and/or the alkaline earth metal to the aqueous solution in order to keep the pH in the specific range defined by the present invention. The temperature of the aqueous solution during soaking is usually 10 to 100°C.

The total amount of the alkali metal and the alkaline earth metal contained in the silica-alumina is usually 0.01 to 3.0 mmols, preferably 0.05 to 2.0 mmols, per 1 g of the silica-alumina.

The soaking is carried out such that the aqueous solution permeates into the central portion of the silica-alumina solids. The soaking is preferably carried out with stirring or circulating the aqueous solution sufficiently during the soaking. In the present invention, a soaking operation must be employed to impregnate the silica-alumina with the aqueous solution. If other methods, such as a spraying method or evaporating to dryness method, are employed, the object of the present invention cannot be achieved.

Next, the second embodiment of the preparation of the catalyst according to the present invention is explained in detail.

The aluminum salt which is decomposed by the heating in order to obtain the carrier is an aluminum salt having a decomposition temperature below 1,500°C. Examples of such aluminum salt include a sulfate, nitrate, halide, organic acid salt and hydroxide of aluminum; a sulfate is preferred. Alumina is not included in the aluminum salt used in the invention. The decomposition temperature does not mean the so-called decomposition temperature described in a literature but means the temperature at which the decomposition of the aluminum salt support in silica set in. Such decomposition temperature in the present invention is, although depending on the purity of the aluminum salt, generally lower than that described in the literature. The decomposition of the aluminum salt, for example aluminum sulfate, can easily be confirmed by evolution of sulfur oxide gas.

Silica can be used in either the form of a gel or sol. Moreover, as silica, either high purity silica prepared from ortho-ethylsilicate or commercially available silica with a small amount of impurity can be used. Although the surface area of the silica is not limited, it is preferably 1 $m^2/g$ or more.

The silica having an aluminum salt supported thereon can be obtained by soaking silica in an aqueous solution containing the aluminum salt or mixing a powdered silica with a powder or slurry of an aluminum salt and then shaping it. The amount of the aluminum salt supported on silica is usually 1 to 50% by weight (as anhydride basis), preferably 5 to 30% by weight.

The decomposition of the aluminum salt is carried out by heating the aluminum salt supported on silica at a temperature higher than the decomposition temperature of the supported aluminum salt. The heating can be carried out in air, nitrogen, carbon dioxide, argon, steam or a mixture thereof. The decomposition temperature is usually below 1,500°C, for example, 100 to 1,200°C. The heating time, which depends on the heating temperature, is usually 0.1 to 24 hours. The occurrence of decomposition as described above, can be confirmed by evolution of a decomposition gas. Thus, the carrier used in the invention can be obtained.

In the second embodiment of the process of preparing a catalyst of the invention, a hydroxide or a weak acid salt of an alkali metal and/or an alkaline earth metal is supported on the above obtained carrier.

Examples of the weak acid are a carbonate, a bicarbonate and an organic acid salt; a hydroxide or a carbonate are preferred. Examples of the alkali metals and the alkaline earth metals are sodium, potassium and magnesium; sodium is preferred.

A hydroxide or a weak acid salt of an alkali metal and/or an alkaline earth metal can be supported on the thus obtained carrier, by spraying an aqueous solution containing a hydroxide or a weak acid of an alkali metal or an alkaline earth metal onto the carrier; putting the carrier into the aqueous solution and evaporating the solution to dryness or soaking the carrier in the aqueous solution and then drying it.

Of those methods, the soaking method is preferable. In that method, from the standpoint of controlling by-production of diisobutylene and the like, the pH of the aqueous solution is kept in the range of 7 to 11, preferably 7 to 10, throughout the soaking. When the pH is too low or too high, controlling of the catalyst activity is sometimes insufficient and consequently the yield of a tertiary olefin becomes low because of increase of undesirable by-products such as a tertiary olefin dimer and a dialkyl ether. Since the pH of the aqueous solution tends to become lower during the soaking, it is preferable to add a solution of an alkali metal and/or an alkaline earth metal in order to keep the pH in the above range.

The temperature of the aqueous solution during soaking is usually 10 to 100°C. The soaking is carried out such that the aqueous solution permeates into the central portion of the carrier. The soaking is preferably carried out with stirring or circulating of the solution sufficiently during the soaking.

The total amount of the alkali metal and the alkaline earth metal contained in the silica-alumina carrier is usually 0.01 to 1.0 mmols, preferably 0.02 to 0.5 mmols, per 1 g of the total weight of silica and the aluminum salt. If the amount is too small, deterioration of the catalyst activity tends to occur too fast.

EP 0 589 557 A2

When the catalyst prepared by either of the aforesaid methods is used in the form of a molded product, molding may be carried out either before or after the soaking. The catalyst thus prepared is preferably calcined before use, and the calcining temperature is usually 150 to 1,000°C, preferably 300 to 700°C.

The process of using a catalyst according to the present invention is explained below.

The process for producing a tertiary olefin according to the invention comprises contacting a gaseous tert-alkyl ether with the catalyst of the first or second embodiment. The process has an advantage that the production reaction proceeds at a relatively low temperature, production of undesirable by-products such as a tertiary olefin dimer and a dialkyl ether is controlled at a low level and the yields of the tertiary olefin which is the desired product and an alcohol which is a useful by-product are high.

Examples of the tert-alkyl ethers are tert-butyl methyl ether, tert-butyl ethyl ether and tert-amyl methyl ether. Examples of the tertiary olefins produced, which depend on the tert-alkyl ether used, are isobutylene, isobutylene and isoamylene, respectively, for the above tert-alkyl ethers. In the present invention, useful alcohols can be obtained as by-products. Examples of the alcohols, which also depend on the tert-alkyl ether used, are methyl alcohol, ethyl alcohol and methyl alcohol, respectively, for the above tert-alkyl ethers.

Although reaction is usually carried out in a catalytic fixed-bed reactor in the gaseous phase, other types of reactors such as a catalytic fluidized bed can be used. The reaction temperature is usually 100 to 400°C, preferably 150 to 300°C. The reaction pressure is usually atmospheric pressure to 20 kg/cm$^2$ (gauge), preferably atmospheric pressure to 10 kg/cm$^2$ (gauge). Although the feed rate of the tert-alkyl ether depends on the reaction temperature, the reaction pressure and the desired conversion of the tert-alkyl ether, it is usually 0.5 to 50 hr$^{-1}$ in terms of LHSV, preferably 1 to 10 hr$^{-1}$. The reaction may be carried out with addition of an inert gas or steam. In particular the production of undesirable by-products such as tertiary olefin dimer or a dialkyl ether can be further decreased by adding water, i.e., steam, to the reaction system. The amount of the water added is usually 0.1 to 20 parts by weight, preferably 0.2 to 10 parts by weight, per the tert-alkyl ether supplied.

The present invention is described in more detail to reference to the following examples.

CATALYST OF FIRST EMBODIMENT

EXAMPLE 1

100 cc of a commercially available silica-alumina (manufacturer: Toyo CCI Co., Ltd., trade name: CS-200, pellets of 5 mmØ x 5 mm H) and 400 ml of pure water were placed in a 500 ml round-bottom flask, and a 1N aqueous sodium hydroxide solution was added thereto to adjust the pH thereof to 8.0 with circulating of the solution. As the pH became lower, the pH was controlled at 7.0 to 8.0 by further adding of aqueous sodium hydroxide solution. The temperature of the mixture was kept at 70°C and the soaking time was 220 hours. The amount of sodium hydroxide contained in the silica-alumina was 0.73 mmol/g. The thus prepared catalyst was then calcined at 500°C for 5 hours, followed by pulverizing to a size of diameter 0.4 to 1 mm. 20 ml of the catalyst was packed in a stainless steel tubular reactor, and tert-butyl methyl ether containing 3% by weight of methanol and 2% by weight of water were supplied thereto at a rate of 60 ml/hr. Reaction was carried out at a temperature of 190°C and a pressure of 5 kg/cm$^2$ (gauge). The reaction product mixture obtained was analyzed. The results obtained are shown in Table 1.

EXAMPLE 2 AND COMPARATIVE EXAMPLES 1 AND 2

Example 1 was repeated except that the conditions shown in Table 1 were used.

COMPARATIVE EXAMPLE 3

Example 1 was repeated except that a catalyst which had not been soaked was used.

EXAMPLE 3

100 cc of the silica-alumina CS-200 was calcined at 800°C for 5 hours with supplying steam (20 g/hr) and nitrogen (50 cc/min) in a flow type furnace. Using thus prepared silica-alumina, Example 1 was repeated except that the sodium hydroxide content in silica-alumina was 0.3 mmol/g and the reaction temperature was 200°C.

4

## COMPARATIVE EXAMPLE 4

Example 3 was repeated except that a catalyst which had not been soaked was used.

The following can be seen from the results.

Examples 1 to 3 show high yield of isobutylene which is the desired product and low yield of undesirable by-products such as diisobutylene and dimethyl ether. On the other hand, Comparative Examples 1 and 2 wherein the pH of the solution used in the soaking is too high, and Comparative Examples 3 and 4 wherein the soaking is not carried out show low yield of isobutylene and high yield of undesirable by-products.

## CATALYST OF SECOND EMBODIMENT

## EXAMPLE 4 AND COMPARATIVE EXAMPLES 5 AND 6

A commercially available silica (manufacturer: Nikki Chemical Co., Ltd., surface area: 100 $m^2/g$, cylindrical pellet of 5 mm$\phi$ $\times$ 5 mm H) was soaked in an aqueous solution containing aluminum sulfate. The amount of aluminum sulfate contained in silica was 10 parts by weight (as anhydride) per 100 parts by weight of silica. The soaked silica was dried and then was heated at 900°C for 5 hours in a nitrogen stream. The catalyst thus prepared was referred to as "catalyst A".

100 cc (78.7 g) of the catalyst A and 500 ml of pure water were placed in a round bottom flask, and the solution was stirred sufficiently by a circulating pump. Thereafter, a 1N aqueous sodium hydroxide solution was added to the mixture to adjust the pH thereof at 8. The total amount of the sodium hydroxide used was 4.0 mg per 1 g of the catalyst. Then, the catalyst was washed with 500 ml of pure water, dried at 150°C for 3 hours, and calcinated at 500°C for 5 hours in air. The catalyst thus prepared was referred to "catalyst B".

Each of the catalyst A and the catalyst B was pulverized to a size of 10 to 24 mesh, and 20 ml of the each catalyst was packed in an each small scale stainless steel tubular reactor (volume of the catalyst bed was 19 mm$\phi$ $\times$ 74 mmH). Tert-butyl methyl ether containing 3% by weight of methanol and 2% by weight of water were supplied to the reactor at a rate of 60 ml/hr (as a volume of liquid, LHSV = 3 $hr^{-1}$), the reaction pressure was 5 $kg/cm^2$ (gauge) and the other reaction conditions were shown in Table 2. The outlet gas was cooled with dry ice-methanol to recover a reaction product and it was analyzed by gas chromatography.

## EXAMPLE 5 AND COMPARATIVE EXAMPLE 7

The reaction was carried out by supplying tert-butyl methyl ether at a rate of 9,460 ml/hr (as a volume of liquid) to a pilot scale reactor packed with the catalyst A (Comparative Example 7) or the catalyst B (Example 5). The LHSV was 2.3 $hr^{-1}$, the reaction pressure was 5 $kg/cm^2$ (gauge) and the reaction temperature was controlled such that the tert-butyl methyl ether conversion was kept at 95%. The resulting temperatures at the time of 100 hours after initiation of the reaction was 206°C for catalysts, whereas the temperature at the time of 700 hours after initiation of the reaction were 219°C for the catalyst B (Example 5) and 230°C for the catalyst A (Comparative Example 7).

The following can be seen from the above results.

Comparative Example 5 wherein the alkali metal of the present invention was not used shows lower selectivity to isobutylene, higher selectivity to diisobutylene and higher selectivity to dimethyl ether as compared with Example 4. Accordingly, Comparative Example 5 is inferior to Example 4 in selective property. Comparative Example 6 wherein the reaction temperature was low shows a similar result to that of Comparative Example 5. Comparative Example 7 shows faster deterioration of the catalyst activity than Example 5, thus needing a higher reaction temperature in order to keep the initial reaction performance. This is not preferable from the viewpoints of process-operability and heat-economy.

## TABLE 1

| | Examples | | |
|---|---|---|---|
| | 1 | 2 | 3 |
| Catalyst preparation condition: | | | |
| pH of aqueous solution for soaking | | | |
| Initial pH of soaking | 8.0 | 10.0 | 9.0 |
| Final pH of soaking | 7.2 | 8.9 | 7.4 |
| Soaking temperature (°C) | 70 | 70 | 70 |
| Soaking time (hr) | 220 | 38 | 20 |
| Reaction condition | | | |
| Reaction temperature (°C) | 190 | 190 | 200 |
| Reaction result[*1]: | | | |
| Conversion of MTBE (%) | 95.5 | 94.6 | 96.5 |
| Selectivity of IB (%) | 99.0 | 98.8 | 99.7 |
| Yield of IB (%) | 94.5 | 93.5 | 96.2 |
| Selectivity of DIB (%) | 1.0 | 1.0 | 0.5 |
| Selectivity of DME (%) | 0.1 | 0.1 | 0.1 |

Table 1 (continued)

| | Comparative Examples | | | |
|---|---|---|---|---|
| | 1 | 2 | 3 | 4 |

Catalyst preparation condition:

pH of aqueous solution for soaking

| | | | | |
|---|---|---|---|---|
| Initial pH of soaking | 12.0 | 13.0 | *2 | *2 |
| Final pH of soaking | 8.2 | 7.4 | − | − |
| Soaking temperature (°C) | 70 | 70 | − | − |
| Soaking time (hr) | 28 | 168 | − | − |

Reaction condition

| | | | | |
|---|---|---|---|---|
| Reaction temperature (°C) | 190 | 190 | 190 | 180 |

Reaction result[1]:

| | | | | |
|---|---|---|---|---|
| Conversion of MTBE (%) | 94.8 | 95.0 | 96.0 | 95.1 |
| Selectivity of IB (%) | 97.4 | 96.7 | 68.9 | 89.0 |
| Yield of IB (%) | 92.3 | 91.9 | 66.4 | 84.6 |
| Selectivity of DIB (%) | 2.5 | 2.6 | 23.5 | 8.4 |
| Selectivity of DME (%) | 0.2 | 0.7 | 1.0 | 0.4 |

7

<u>TABLE 2</u>

| | Example | Comparative Examples | |
|---|---|---|---|
| | 4 | 5 | 6 |
| Reaction condition: | | | |
| Catalyst[3] | B | A | A |
| Reaction temperature (°C) | 200 | 200 | 190 |
| Reaction result[1]: | | | |
| Conversion of MTBE (%) | 95.4 | 97.2 | 95.0 |
| Selectivity of IB (%) | 99.6 | 97.6 | 99.1 |
| Yield of IB (%) | 95.0 | 94.9 | 94.1 |
| Selectivity of DIB (%) | 0.32 | 2.30 | 0.87 |
| Selectivity of DME (%) | 0.09 | 0.14 | 0.10 |

Notes:

[1]

| | |
|---|---|
| MTBE: | tert-Butyl methyl ether |
| Conversion of MTBE: | Reacted MTBE/supplied MTBE×100% |
| IB: | Isobutylene |
| Selectivity of IB: | Produced IB (mole)/reacted MTBE (mole)×100% |
| Yield of IB: | Produced IB (mole)/supplied MTBE (mole)×100% |
| DIB: | Diisobutylene |
| Selectivity of DIB: | Produced DIB (mole)/reacted MTBE (mole)×100% |
| Selectivity of DME: | Produced DME (mole)/reacted |

MTBE (mole)×100%

*2

In Comparative Examples 3 and 4, soaking was not carried out.

*3

A: Catalyst without a hydroxide or a weak acid salt of an alkali metal or an alkaline earth metal of the present invention

B: Catalyst prepared according to the present invention

As described above, the present invention provides a process for producing a tertiary olefin and a catalyst suitably used in the process, wherein the process has advantages that the production reaction proceeds at a relatively low temperature, production of undesirable by-products such as a tertiary olefin dimer and a dialkyl ether is controlled at low level and the yields of the tertiary olefin which is the desired product and an alcohol which is a useful by-product are high.

## Claims

1. A process of preparing a silicon-aluminum catalyst which comprises soaking silica-alumina in an aqueous solution of a hydroxide or a weak acid salt of an alkali metal and/or an alkaline earth metal, and maintaining the aqueous solution at a pH in the range of 7 to 11 throughout the soaking.

2. A process according to Claim 1, wherein the silica-alumina is calcined at 700 to 1,200°C before the soaking.

3. A process of preparing a silicon-aluminum catalyst which comprises contacting a hydroxide or a weak acid salt of an alkali metal and/or an alkaline earth metal with a carrier which was obtained by heating silica having an aluminum salt supported thereon at a temperature higher than the temperature at which said aluminum salt decomposes.

4. A process according to Claim 3, wherein the aluminum salt is a sulfate, preferably of aluminium, a nitrate, a halide, an organic acid salt or a hydroxide of aluminum.

5. A process according to Claim 3, wherein the decomposition of the aluminum salt is carried out by heating the aluminum salt supported on silica at a temperature of 100 to 1,200°C in air, nitrogen, carbon dioxide, argon or steam.

6. A process according to Claim 3, 4 or 5, wherein the carrier is soaked in an aqueous solution of an alkali metal and/or an alkaline earth metal whilst the pH of the aqueous solution is kept in the range of 7 to 11 throughout the soaking.

7. A process according to any of Claims 1 to 6, wherein the weak acid is a carbonate.

8. A process according to any of Claims 1 to 6, wherein the alkali metal is sodium or potassium.

9. A process according to any of Claims 1 to 6, wherein the alkaline earth metal is magnesium.

**10.** A process of producing a tertiary olefin, which comprises contacting a gaseous tert-alkyl ether with a catalyst prepared by a process as claimed in any preceding claim.

**11.** A process according to Claim 10, wherein the tert-alkyl ether is tert-butyl methyl ether, tert-butyl ethyl ether or tert-amyl methyl ether.

**12.** A process according to Claim 10 or 11, wherein the reaction is carried out with addition of steam, preferably in amount of 0.1 to 20 parts by weight of steam per the tert-alkyl ether supplied.